(19) Europäisches Patentamt
European Patent Office
Office européen des brevets

(11) Veröffentlichungsnummer: **0 638 556 A1**

# (12) EUROPÄISCHE PATENTANMELDUNG

(21) Anmeldenummer: **94111858.0**

(22) Anmeldetag: **29.07.94**

(51) Int. Cl.[6]: **C07D 231/20**, A61K 31/415, C07D 231/28, C07D 231/48, C07D 231/54, C07D 405/04, C07D 495/04

(30) Priorität: **11.08.93 DE 4326904**
**28.04.94 DE 4414792**

(43) Veröffentlichungstag der Anmeldung:
**15.02.95 Patentblatt 95/07**

(84) Benannte Vertragsstaaten:
**AT BE CH DE DK ES FR GB GR IE IT LI LU MC NL PT SE**

(71) Anmelder: **BAYER AG**

**D-51368 Leverkusen (DE)**

(72) Erfinder: **Wachtler, Peter, Dr.**
**Morgengraben 4**
**D-51061 Köln (DE)**
Erfinder: **Heuer, Lutz, Dr.**
**Scheiblerstrasse 83**
**D-47800 Krefeld (DE)**
Erfinder: **Sperzel, Michael, Dr.**
**Normannenstrasse 31**
**D-42275 Wuppertal (DE)**
Erfinder: **Stünkel, Klaus Georg, Dr.**
**Am Eckbusch 55**
**D-42113 Wuppertal (DE)**

(54) **Arzneimittel enthaltend 1-Thiocarbamoyl-5-hydroxypyrazolen und deren Verwendung als Mittel zur Bekämpfung des septischen Schocks.**

(57) Die Erfindung betrifft die Verwendung von 1-Thiocarbamoyl-5-hydroxypyrazolen der allgemeinen Formel

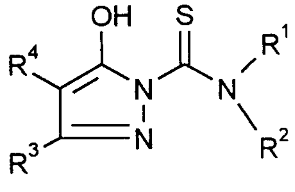

(I)

wobei die Substituenten die in der Beschreibung benannten Reste bedeuten, als pharmazeutische Mittel zur Bekämpfung des septischen Schocks und der damit verbundenen Folgen.

EP 0 638 556 A1

Die Erfindung betrifft die Verwendung von 1-Thiocarbamoyl-5-hydroxypyrazolen der allgemeinen Formel

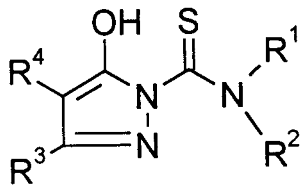

(I)

wobei die Substituenten die in den Ansprüchen benannten Reste bedeuten, als pharmazeutische Mittel zur Bekämpfung des septischen Schocks und der damit verbundenen Folgen.

Thiocarbamoylpyrazole stellen eine Substanzklasse dar, die im Materialschutz bekannt ist, da zahlreiche Vertreter dieses Strukturtyps mikrobizide Eigenschaften aufweisen und somit wertvolle Materialschutzmittel darstellen, beispielsweise zum Schutz von Farben oder Holz. Diese Anwendung ist beispielsweise in der EP 515 934 beschrieben. Dagegen ist die in der vorliegenden Erfindung beanspruchte Verwendung von Thiocarbamoylpyrazolen im pharmazeutischen Bereich, insbesondere zur Behandlung des septischen Schocks, bislang nicht beschrieben worden.

Die Erfindung betrifft somit Arzneimittel, enthaltend Verbindungen der allgemeinen Formel (I)

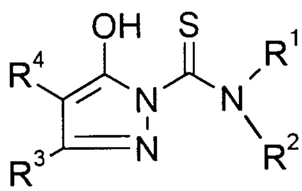

(I)

in der

$R^1$, $R^2$ unabhängig voneinander für Wasserstoff, Alkyl, Cycloalkyl, Alkenyl, Aralkyl oder Aryl stehen, letzteres gegebenenfalls substituiert mit $C_{1-6}$-Alkyl oder Halogen

oder für $R^1$ = H, $R^2$ = $NH_2$

$R^3$, $R^4$ unabhängig voneinander für Wasserstoff oder gegebenenfalls substituiertes Alkyl, Alkenyl, Alkinyl, Cycloalkyl, Cycloalkenyl, Alkyl(cycloalkyl), Alkenyl(cycloalkenyl), Alkoxy, Alkylthio, Aralkoxy, Aralkylthio, Aralkyl, Aryl, Hetaryl, Aryloxy, Hetaryloxy, Arylthio, Hetarylthio, Alkoxycarbonyl, Alkoxycarbonalalkyl, Cyanoalkyl oder

$R^3$, $R^4$ bilden einen gesättigten oder teilweise oder gänzlich ungesättigten gegebenenfalls substituierten Ring, in Summe von 5 bis 9 Atomen, wovon alle bis auf maximal zwei Stickstoff-, Schwefel- oder Sauerstoffatome Kohlenstoffatome sind, oder ihre Metallkomplexe mit Metallen wie z.B. Eisen, Zink, Mangan, Calcium, Magnesium, Kalium, Natrium, Cithium, Canthon, Platin, Gold und Aluminium sowie ihre physiologisch vertretbaren Salze mit Säuren wie HCl, $HNO_3$, Essigsäure, Salicylsäure etc.

Substituiert bedeutet in den Definitionen für $R^3$, $R^4$: mit ein bis sieben, aber maximal soviele wie H-Atome im unsubstituierten Fall enthalten sind, Atome oder Atomgruppen, ausgewählt aus: F, Cl, Br, I, $NO_2$, CN, O-Alyl, S-Alyl, Alkyl, - $CO_2$-Alkyl, Aryl, $NO_2$.

Folgende Definitionen stehen hier und im folgenden

Alkyl = geradkettiges oder verzweigtes Alkyl mit 1 bis 18 C-Atomen, wie Me, Et, n-, i-Propyl, N-, i-, s- und tert.-Butyl, n-, i und tert.-Pentyl, n-Hexyl, n-Octyl, n-Nonyl, n-Decyl, n-Undecyl, n-Dodecyl, n-Tridecyl, n-Tetradecyl, n-Pentadecyl, n-Hexadecyl, n-Heptodecyl oder n-Octadecyl oder ihre verzweigten Strukturisomeren.

Diese Alkylgruppen können durch 1 bis 15 Halogenatome, vorzugsweise Chlor und/oder Fluor oder CN substituiert sein.

Auch kann die Kette von C-Atomen durch 1 bis 2 Heteroatomen wie Sauerstoff oder Schwefel, oder Atomgruppen wie N-Me, N-Et, -S(O), $-SO_2$ unterbrochen sein, ohne das sich seine Gesamtanzahl an Atomen ändert.

Alkenyl (+ Alkinyl) = ist definiert wie Alkyl, nur insofern geändert, daß mindestens eine und maximal drei C-C-Einfachbindung durch eine C-C-Doppel(Dreifach)bindung ersetzt wurde. Die Anzahl an C-Atomen beträgt mindestens drei und wird mit jeder weiteren Doppelbindung (Dreifachbindung), die hinzukommt um mindestens zwei C-Atome verlängert.

Cycloalkyl- und Cycloalkenylgruppen umfassen Cycloalkyl mit vorzugsweise 3 (5) bis 7 C-Atomen, wie beispielsweise Cyclopropyl, Cyclobutyl, Cycloheptyl, Cyclopentyl, Cyclopentenyl, Cyclohexenyl, Cyclohexyl; bevorzugte substituierte Cycloalkylgruppen umfassen durch 1 bis 3 $C_1$-$C_4$-Alkylgruppen oder 1 bis 3 Halogenatomen, wie Chlor und/oder Fluor, substituiertes Cycloalkyl, wie beispielsweise Methylcyclohexyl, Dimethylcyclohexyl, 1,3,3-Trimethylcyclohexyl, 3-Chlorcyclohexyl. Alkyl(cycloalkyl)- [und Alkyl-(cycloalkenyl)-Gruppen] enthalten vorzugsweise 1 bis 6 C-Atome im geradkettigen oder verzweigten Alkylteil und 3 bis 7 -Atome im Cycloalkyl/alkenyl-Teil; besonders (1-Cyclopentyl)methyl, (1-Cyclopentenyl)-methyl, (1-Cyclohexenyl)methyl, (1-Cyclohexyl)methyl, (1-Cyclopropyl)methyl.

Alkoxycarbonyl steht für geradkettiges oder verzweigtes Alkoxycarbonyl mit vorzugsweise 1 bis 6 C-Atomen im Alkoxyrest, wie beispielsweise Methoxycarbonyl, Ethoxycarbonyl, n- und i-Propoxycarbonyl, n-, i-, sek.- und tert.-Butoxycarbonyl, Hexoxycarbonyl. Analoges gilt für die Alkoxycarbonylalkylgruppen.

Aralkyl enthält vorzugsweise 1 bis 6, insbesondere 1 bis 4 C-Atome im geradkettigen oder verzweigten Alkylteil und vorzugsweise Phenyl oder Naphthyl als Arylteil. Beispiele für solche Aralkylgruppen umfassen Benzyl, $\alpha$-Methylbenzyl, $\alpha,\alpha$-Dimethylbenzyl, 2-Phenethyl, $\alpha$- und $\beta$-Naphthylmethyl. Diese Aralkylreste können 1 bis 3 Substituenten aus der Reihe Halogen (insbesondere Chlor und/oder Fluor), Nitro, Cyano, gegebenenfalls halogeniertes $C_1$-$C_4$-Alkyl oder -Alkoxy, wie beispielsweise Methyl, Ethyl, Trifluormethyl, Difluorchlormethyl, Difluormethyl, Trichlormethyl, Methoxy, Ethoxy, Trifluormethoxy, Difluorchlormethoxy und Difluormethoxy, gegebenenfalls halogeniertes $C_1$-$C_4$-Alkylmercapto, wie beispielsweise Methylmercapto, Trifluormethylmercapto, Difluorchlormethylmercapto tragen.

Unter dem Begriff Aryl ist unsubstituiertes oder substituiertes Aryl mit vorzugsweise 6 bis 12 C-Atomen im Arylteil zu verstehen. Bevorzugte Beispiele umfassen Phenyl, Biphenyl und Naphthyl. Die Arylgruppen können 1 bis 3 Substituenten aus der Reihe Halogen (insbesondere Chlor und/oder Fluor), $C_1$-$C_6$-Alkyl, -Alkoxy oder Thioalkoxy, Halogen-$C_1$-$C_2$-alkyl (wie Trifluormethyl, Difluormethyl), Cyano, Nitro, $C_1$-$C_6$-Alkoxycarbonyl oder Amino tragen.

Unter dem Begriff Alkoxy ist geradkettiges und verzweigtes Alkoxy mit vorzugsweise 1 bis 12, insbesondere 1 bis 4 C-Atomen zu verstehen. Bevorzugte Beispiele umfassen Methoxy, Ethoxy, n- und i-Propoxy, n-, i-, sek.- und ter.-Butoxy, Pentoxy, Hexoxy, Heptoxy, Octoxy, Nonoxy und Decoxy. Die Alkoxygruppen können durch 1 bis 3 Halogenatome (Cl, F) substituiert sein, bevorzugt: O-$CF_3$, O-$CHF_2$, O-$CF_2$-O, O-$CF_2$-$CF_2$-O, O-CFCl-CFCl-O-.

Alkylthio steht für geradkettiges oder verzweigtes Alkylthio mit vorzugsweise 1 bis 12 C-Atomen. Bevorzugte Beispiele umfassen Methylthio, Ethylthio, n- und i-Propylthio, n-, i-, sek.- und ter.-Butylthio, n-Pentylthio und seine Isomeren wie 1-, 2- und 3-Methyl-butylthio. Die Alkylthiogruppen können durch 1 bis 3 Halogenatome (vorzugsweise Chlor und/oder Fluor) substituiert sein; bevorzugte Beispiele hierfür sind Di- und Trifluormethylthio sowie Difluorchlormethylthio.

Aralkoxy enthält vorzugsweise 1 bis 6 C-Atome im geradkettigen oder verzweigten Alkylteil und vorzugsweise Phenyl als Arylteil. Bevorzugte Beispiele sind Benzyloxy und Phenethyloxy. Die Aralkoxygruppen können durch 1 bis 3 Halogenatome (vorzugsweise Chlor und/oder Fluor) oder durch eine $C_1$-$C_4$-Alkylgruppe substituiert sein.

Cyanoalkyl wie Alkyl (1 bis 6) nur mit Cyanosubstituiert, bevorzugt endständig.

Hetaryl: Furanyl, Thienyl, Thiazolyl, Pyrazolyl, Pyrrolyl, Imidazyl, Triazolyl, gegebenenfalls mit 1 bis 2 Halogen oder Alkyl, Alkoxy oder Thioalkoxy-Substituenten.

Halogen: F, Cl, Br, I.

Aralkylthio enthält vorzugsweise 1 bis 6 C-Atome im geradkettigen oder verzweigten Alkylteil und vorzugsweise Phenyl als Arylteil. Bevorzugtes Beispiel ist Benzylthio. Die Aralkylthiogruppen können durch 1 bis 3 Halogenatome (vorzugsweise Chlor und/oder Fluor) oder durch eine $C_1$-$C_4$-Alkylgruppe substituiert sein.

Aryloxy enthält vorzugsweise 1 bis 10 C-Atome im Arylteil. Bevorzugte Beispiele sind Phenoxy und Naphthoxy. Die Aryloxygruppen können durch 1 bis 3 Substituenten aus der Reihe Halogen (vorzugsweise Chlor und/oder Fluor), $C_1$-$C_4$-Alkyl, Halogen-$C_1$-$C_2$-alkyl (wie Di- und Trifluormethyl), Cyano, Nitro oder Amino tragen.

Arylthio enthält vorzugsweise 6 bis 10 C Atome im Arylteil. Bevorzugte Beispiele sind Phenylthio und Naphthylthio. Die Arylthiogruppen können die unter "Aryloxy" aufgezählten Substituenten tragen.

Bevorzugte Beispiele für 1,$\omega$-$C_3$-$C_6$-Alk(en)ylenreste umfassen 1,3-Propylen, 1,4-Butylen und 1,4-Butadien(1,3)ylen.

Verbindungen der Formel (I), worin

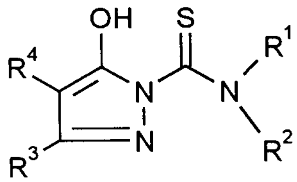

(I)

$R^1$ Wasserstoff oder gegebenenfalls substituiertes Alkyl, Alkenyl oder Aryl,
$R^2$ Wasserstoff
$R^3$ Wasserstoff oder gegebenenfalls substituiertes $C_1$-$C_8$-Alkyl, Cycloalkyl, Alkenyl, Aralkyl oder Aryl
$R^4$ Wasserstoff oder gegebenenfalls substituiertes Alkyl, Cycloalkyl, Alkenyl, Aralkyl oder Aryl bedeuten, sind bevorzugt.

Verbindungen der Formel (I), worin
$R^1$, $R^2$ Wasserstoff
$R^3$ gegebenenfalls substituiertes $C_1$-$C_6$-Alkyl, Aralkyl oder Aryl
$R^4$ Wasserstoff, gegebenenfalls substituiertes Alkyl, Aralkyl oder Aryl bedeuten, sind besonders bevorzugt.

Verbindungen der Formel (I), worin
$R^1$ und $R^2$ Wasserstoff,
$R^3$ gegebenenfalls substituiertes $C_1$-$C_6$-Alkyl oder Aralkyl
$R^4$ Wasserstoff, gegebenenfalls substituiertes $C_1$-$C_6$-Alkyl, Aralkyl
bedeuten, sind ganz besonders bevorzugt.

Die erfindungsgemäß zu verwendenden Verbindungen I können als verschiedene (s.u.) Tautomere vorliegen, u.a. auch in ihrer tautomeren Pyrazol-5-on-Form.

Die erfindungsgemäß zu verwendenden Verbindungen I und Verfahren zu ihrer Herstellung sind bekannt. So werden in den veröffentlichten japanischen Patentanmeldungen 79/115 374 und 79/119 031 3-mono und 3,4-disubstituierte 1-Thiocarbamoyl-5-hydroxy-pyrazole beschrieben, die gegen Pflanzenkrankheiten wirksam sind. Insbesondere sollen sie fungizide Wirkung besitzen; vgl. auch J. Pesticide, Sci. 11, 205-212 (1986).

Aus Arch. Pharm. 316 (1983) 2-6 und aus Sci. Pharm. 51 (2) (1982) 167-172 sind 4-mono- und 3,4-disubstituierte 1-Thiocarbamoyl-5-hydroxy-pyrazole bekannt, die als Zwischenprodukte zur Herstellung von Präparaten mit antihistaminischer Wirkung dienen.

In der EP 515 934 ist die Verwendung der 1-Thiocarbamoyl-5-hydroxypyrazole als Materialschutzmittel beansprucht.

Soweit die Verbindungen noch neu sind, können sie analog den bekannten Herstellungsverfahren hergestellt werden. Üblicherweise setzt man einen α-Formylessigsäureester oder ein α-Formylessigsäureamid oder ein β-Ketoessigsäureester oder ein β-Ketoessigsäureamid mit einem gegebenenfalls substituierten Thiosemicarbazid um. Diese Kondensationsreaktion verläuft nach folgendem Reaktionsschema (demonstriert am β-Ketoessigsäureethylester als β-Diketo-Ausgangsprodukt):

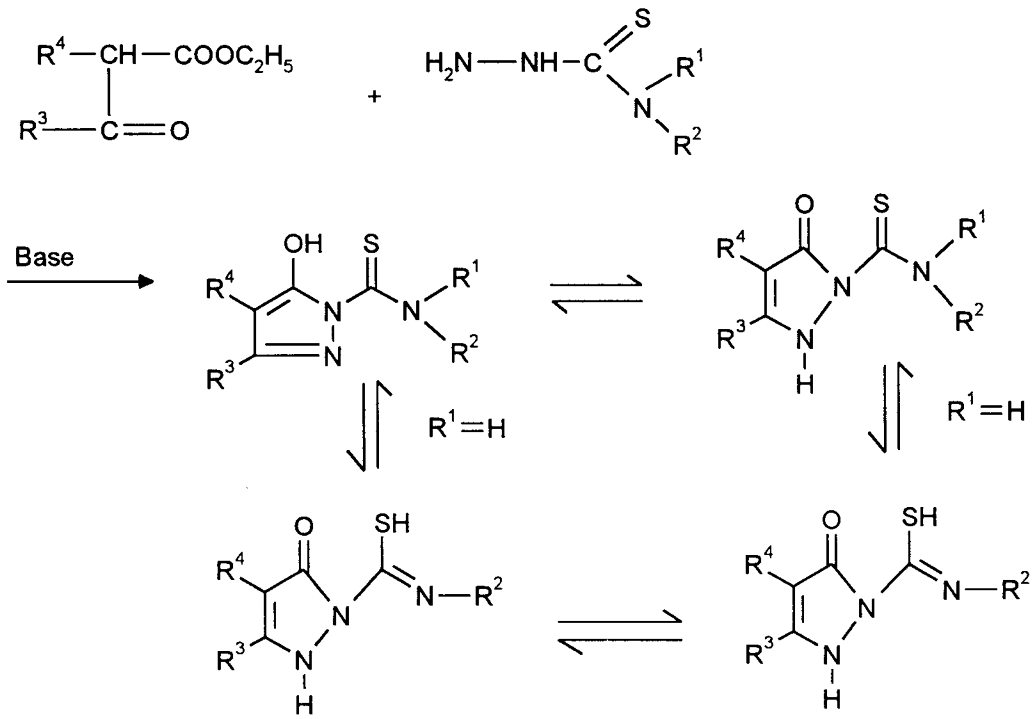


Die Substituenten $R^1$ bis $R^4$ haben im obigen Reaktionsschema die oben zu den Verbindungen der Formel I angegebenen Bedeutungen.

Pro Mol $\alpha$-Formylessigsäurederivat bzw. pro Mol $\beta$-Diketoverbindung setzt man vorzugsweise 0,8 bis 1,0 Mol Thiosemicarbazid zu.

Zur Erleichterung der Ringschlußreaktion setzt man vorteilhafterweise Basen wie Natriumhydroxid, Kaliumhydroxid oder Kalium-tert.-butylat zu. Vorzugsweise setzt man die Base in einer dem $\alpha$-Formylessigsäurederivat bzw. der $\beta$-Diketoverbindung etwa äquivalenten Menge zu.

Die Kondensation kann gegebenenfalls in Gegenwart eines Lösungsmittels durchgeführt werden; als Lösungsmittel haben sich vor allem Alkohole wie Ethanol oder aromatische Kohlenwasserstoffe wie Toluol bewährt.

Die Kondensationsreaktion ist innerhalb eines größeren Temperaturbereichs durchführbar. Für die zuerst ablaufende Thiosemicarbazonbildung kann bei Temperaturen von 20 bis 110 °C gearbeitet werden, vorzugsweise zwischen 60 und 90 °C. Die nach der Basenzugabe ablaufende Cyclokondensationsreaktion kann bei Temperaturen von 20 bis 100 °C, vorzugsweise 20 bis 40 °C, vorgenommen werden. Da die Basenzugabe in manchen Fällen exotherm verläuft, kann ein Kühlen bei diesem Reaktionsschritt erforderlich sein.

Die 1-Thiocarbamoyl-5-hydroxy-pyrazole können nach bekannten Methoden aus den Reaktionsgemischen isoliert werden. Man geht im allgemeinen so vor, daß man die Reaktionsgemische vom Lösungsmittel befreit und den Rückstand mit wäßriger Salzsäure behandelt. Die dabei ausfallenden Pyrazole werden durch Absaugen abgetrennt. Es ist jedoch auch möglich, das Reaktionsgemisch unmittelbar in einen großen Überschuß verdünnter Salzsäure einzugießen und die als Niederschlag sich abscheidenden Pyrazole abzufiltrieren.

Die für die Herstellung der erfindungsgemäß zu verwendenden Verbindungen I benötigen Ausgangsverbindungen, nämlich die $\alpha$-Formylessigsäureester bzw. - amide und die gegebenenfalls in $\alpha$-Stellung substituierten $\beta$-Ketoessigsäureester bzw. -amide und die gegebenenfalls substituierten Thiosemicarbazide, sind entweder bekannte Verbindungen oder in Analogie zu bekannten Verbindungen nach vorbeschriebenen Verfahren herstellbar. Hierbei sind folgende Methoden hilfreich: Tietze, Eicher, Reaktionen und Synthesen, Thieme Verlag 1981, S. 153, 440, Oikawa, J. Org. Chem., 43, 2087 (1978).

Wie oben bereits gesagt, sind manche erfindungsgemäß zu verwendenden Verbindungen I neu. Weiterer Gegenstand der Erfindung sind daher Verbindungen der Formel (II)

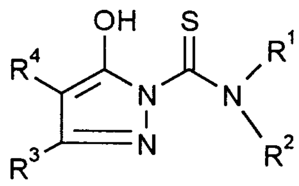 (II)

worin

$R^1$, $R^2$ = H, Alkenyl oder Aryl, mit der Bedingung, daß $R^1$ und $R^2$ nicht gleichzeitig Wasserstoff sind, bedeuten und

$R^3$, $R^4$ die oben zu den Verbindungen der Formel (I) angegebenen Bedeutungen aufweisen.

**Material und Methoden**

**Endotoxischer Schock**

$B_6D_2F_1$ Mäuse werden vorbehandelt mit Galktosamin (600 mg/kg) und mit LPS (S.arbortus equi, 0,01 µg/mous) der endotoxische Schock ausgelöst. Die Mäuse sterben 8 bis 24 Stunden nach LPS-Gabe. Die Substanzen werden intravenös, subkutan, intraperitoneal oder per oral eine Stunde vor LPS-Gabe appliziert.

**TNFα-Aktivität im Serum**

NMRI-Mäuse werden mit den Substanzen eine Stunde vor LPS-Gabe behandelt. 1,5 Stunden nach Auslösen des Schockes werden Blutproben entnommen, daraus Serum gewonnen und diese bis zur Testung bei -70 °C eingefroren. Die TNFα-Serumaktivität wird nach einer modifizierten Methode von Espevik et al. (J. Immunol.Methods 956:99, 1986), die auf der Zytolyse TNFα-empfindlicher Zellen (WEHI-164/13) basiert, bestimmt: $5x10^4$ Zellen werden in 96-Lochplatten in FCS-RPMI-Medium übernacht inkubiert. Danach werden die Zellen mit den Serumproben in verschiedenen Konzentrationen inkubiert (übernacht bei 37 °C und 7 % $CO_2$). Als Kontrolle werden Seren von unbehandelten oder nur mit LPS behandelten Mäusen eingesetzt. Nach den Inkubationsschritten werden die Zellen gefärbt.

**Wirkung von Thiocarbamoylpyrazolonen**

Die Wirkung von Thiocarbamoylpyrazolonen wurde nach einer einmaligen intravenösen, subkutanen, intraperitonealen oder oralen Gabe gezeigt.

| Beispiel | Hemmung des endotoxischen Schocks in Mäusen (Letalität) |
|---|---|
| 2/005 | |
| 2/007 | |
| 2/011 | |
| 2/012 | ≦10 mg/kg iv |
| 2/013 | |
| 3/035 | |
| 4/004 | |
| 10/007 | |

Die Prävention der Letalität durch die Thiocarbamoylpyrazolone korreliert mit der Hemmung der TNFα-Aktivität im Serum (Beispiele: 3/035 und 2/036 bei 10 mg/kg).

## Beispiele

Nr. 7 aus Tabelle 1(1/007)

10,3 g (0,06 Mol) α-Formyl-hexansäureethylester und 5,5 g (0,06 Mol) Thiosemicarbazid werden in 200 ml Ethanol vorgelegt und 3 Stunden bei 80 °C gerührt.

Dann wird auf Raumtemperatur gebracht und unter Rühren 6,9 g Kalium-tert.-burylat zugegeben, um danach 4 Stunden bei Raumtemperatur weiterzurühren. Anschließend wird der Kolbeninhalt in ein Gemisch aus 800 ml Wasser und 50 ml conc. Salzsäure eingerührt und der erhaltene Niederschlag abgesaugt. Nach gründlichem Waschen mit Wasser wird das Produkt im Trockenschrank bis zur Gewichtskonstanz belassen. Durch Umkristallisieren aus Ethanol kann die Verbindung gereinigt werden.

Ausbeute: 9,4 g (80,3 % d. Th.)
Fp.: 139-141 °C

In gleicher Weise können aus den entsprechenden α-Formyl-carbonsäureestern die in der nachfolgenden Tabelle 1 aufgeführten, 4-substituierten 1-Thiocarbamoyl-5-hydroxypyrazole erhalten werden.

## Tabelle 1

$R^1 = R^2 = R^3 = H$

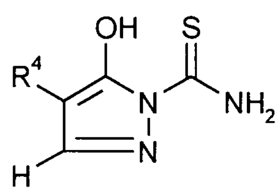

| Bsp.-Nr. | $R^4$ | Fp. |
|---|---|---|
| 1 | H- | |
| 2 | Me- | 164-165°C |
| 3 | Et- | 144-145°C |
| 4 | n-Pr- | 146-147°C |
| 5 | i-Pr- | |
| 6 | c-Pr- | |
| 7 | n-Bu- | 139-141°C |
| 8 | i-Bu | 128-131°C |
| 9 | s-Bu- | |
| 10 | t-Bu- | |
| 11 | n-$C_5H_4$ | |
| 12 | 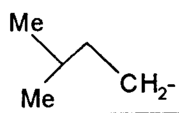 | 153-154°C |
| 13 | 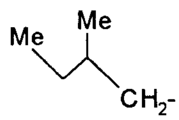 | |
| 14 | 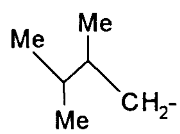 | |
| 15 | 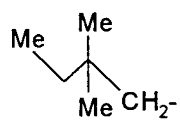 | |
| 16 | 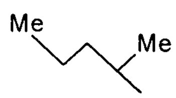 | |
| 17 | 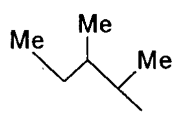 | |
| 18 | 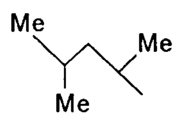 | |

| Bsp.-Nr. | $R^4$ | Fp. |
|---|---|---|
| 19 | 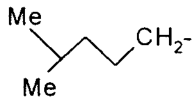 | |
| 20 | 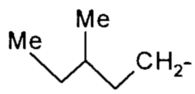 | |
| 21 | 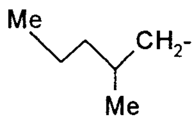 | |
| 22 | 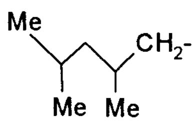 | |
| 23 | 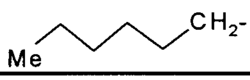 | 136-137°C |
| 24 | 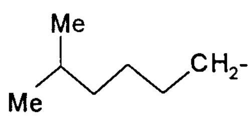 | |
| 25 | 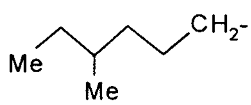 | |
| 26 | 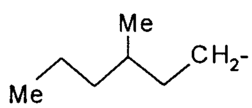 | |
| 27 | 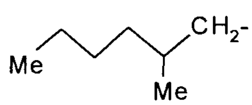 | |
| 28 | 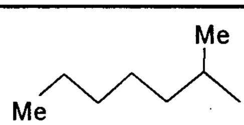 | |
| 29 | 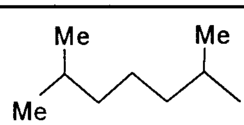 | |
| 30 | 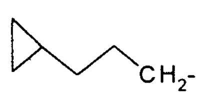 | |
| 31 | 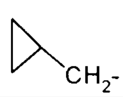 | |

| Bsp.-Nr. | $R^4$ | Fp. |
|---|---|---|
| 32 | 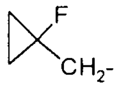 | |
| 33 | 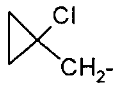 | |
| 34 | 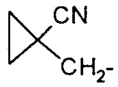 | |
| 35 | $NC-CH_2-CH_2-$ | |
| 36 | $CF_3-$ | |
| 37 | $CF_3-CF_2-$ | |
| 38 | $CF_3-CF_2-CF_2-$ | |
| 39 | $CF_2-CF_2-CH_2-$ | |
| 40 | $CF_3-CF_2-CF_2-CH_2-$ | |
| 41 | 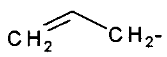 | 134-135°C |
| 42 | 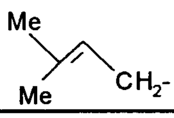 | |
| 43 | 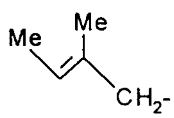 | |
| 44 | 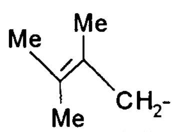 | |
| 45 | 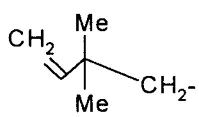 | |
| 46 | 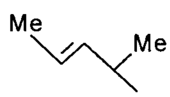 | |
| 47 | 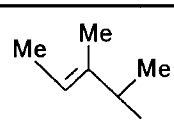 | |

| Bsp.-Nr. | $R^4$ | Fp. |
|---|---|---|
| 48 | 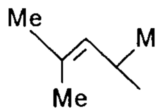 | |
| 49 | 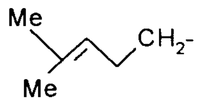 | |
| 50 | 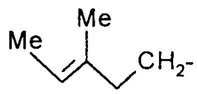 | |
| 51 | 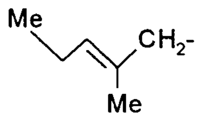 | |
| 52 | 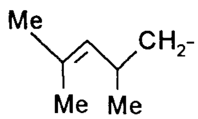 | |
| 53 | 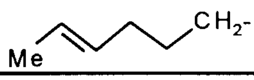 | |
| 54 | 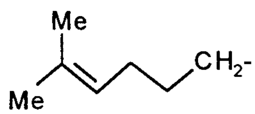 | |
| 55 | 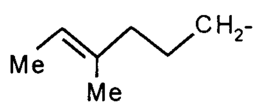 | |
| 56 | 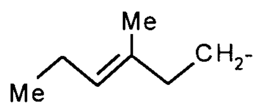 | |
| 57 | 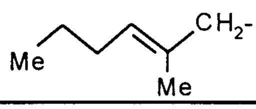 | |
| 58 | 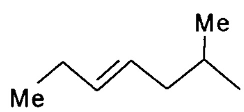 | |
| 59 | 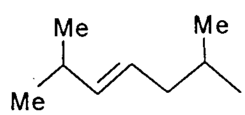 | |

| Bsp.-Nr. | $R^4$ | Fp. |
|---|---|---|
| 60 | 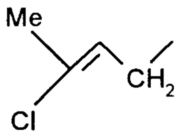 | |
| 61 | 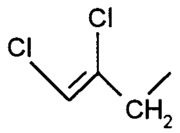 | |
| 62 | 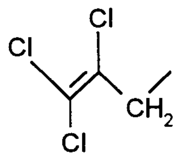 | |
| 63 | 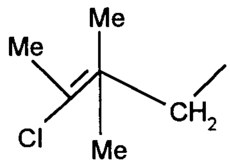 | |
| 64 | 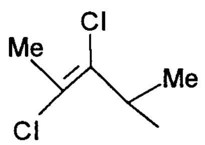 | |
| 65 | 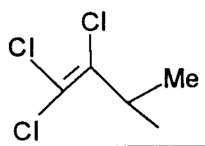 | |
| 66 | 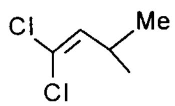 | |
| 67 | 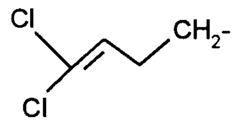 | |
| 68 | 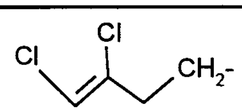 | |
| 69 | 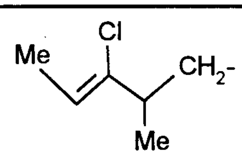 | |

| Bsp.-Nr. | $R^4$ | Fp. |
|---|---|---|
| 70 | 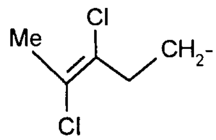 | |
| 71 | 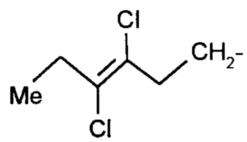 | |
| 72 | 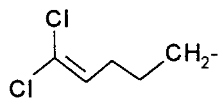 | |
| 73 | 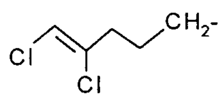 | |
| 74 | 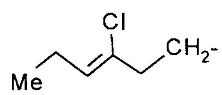 | |
| 75 | 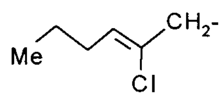 | |
| 76 | 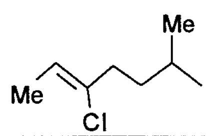 | |
| 77 | 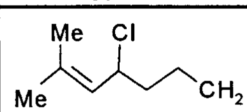 | |
| 78 | n-$C_7H_{15}$ | |
| 79 | n-$C_8H_{17}$ | |
| 80 | n-$C_9H_{19}$ | |
| 81 | n-$C_{10}$-$H_{21}$ | 128-131°C |
| 82 | n-$C_{11}H_{23}$ | |
| 83 | n-$C_{12}H_{25}$ | |
| 84 | n-$C_{13}H_{27}$ | |

| Bsp.-Nr. | $R^4$ | Fp. |
|---|---|---|
| 91 | 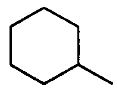 | 157-158°C |
| 92 | 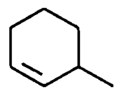 | |
| 93 | 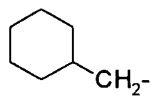 | 185°C |
| 94 | 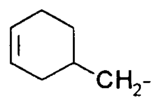 | |
| 95 | 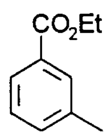 | 165-167°C |
| 96 | 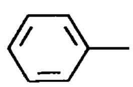 | 176°C |
| 97 | 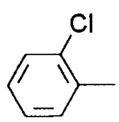 | 231-236°C |
| 98 | 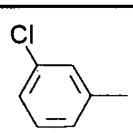 | 168-169°C |
| 99 | 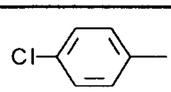 | 231-232°C |

| Bsp.-Nr. | $R^4$ | Fp. |
|---|---|---|
| 100 | 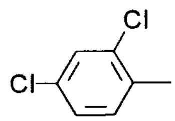 | 171-173°C |
| 101 | 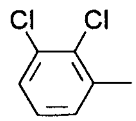 | |
| 102 | 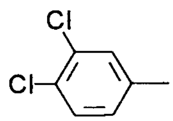 | |
| 103 | 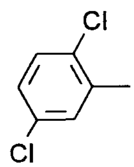 | |
| 104 | 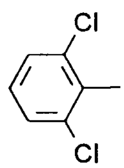 | |
| 105 | 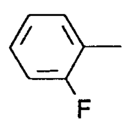 | 157-158°C |
| 106 | 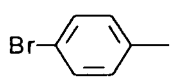 | 194-195°C |
| 107 | 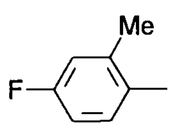 | 171-172°C |
| 108 | 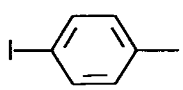 | |
| 109 | 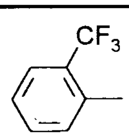 | 174-175°C |

| Bsp.-Nr. | $R^4$ | Fp. |
|---|---|---|
| 110 | 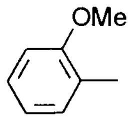 | 150°C |
| 111 | 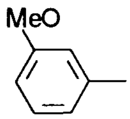 | 162-163°C |
| 112 | 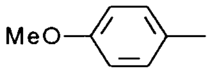 | 240-242°C |
| 113 | 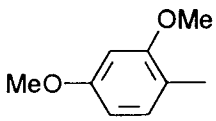 | |
| 114 | 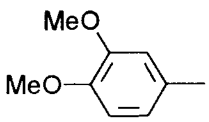 | 263-265°C |
| 115 | 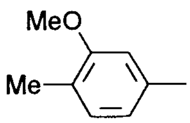 | 167°C |
| 116 | 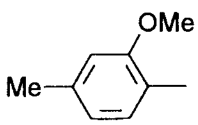 | |
| 117 | 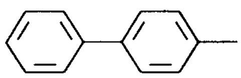 | 284-286°C |
| 118 | 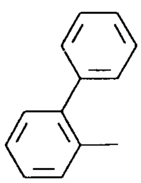 | |
| 119 | 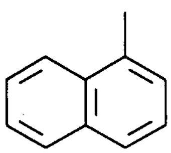 | 174-176°C |
| 120 | 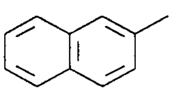 | 236-237°C |

| Bsp.-Nr. | $R^4$ | Fp. |
|---|---|---|
| 121 | 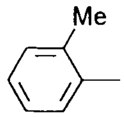 | 200°C Zers. |
| 122 | 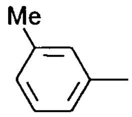 | 177°C |
| 123 | 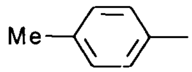 | 197-198°C |
| 124 | 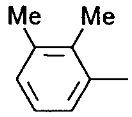 | |
| 125 | 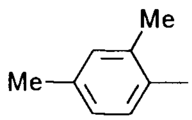 | 178-179°C |
| 126 | 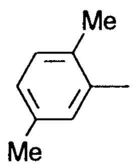 | |
| 127 | 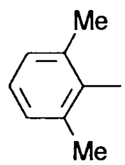 | |
| 128 | 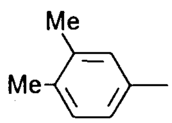 | |
| 129 | 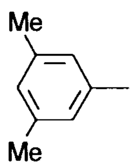 | |
| 130 | 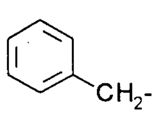 | 161-162°C |

| Bsp.-Nr. | $R^4$ | Fp. |
|---|---|---|
| 131 | 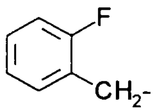 | 170-172°C |
| 132 | 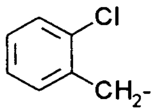 | 152-154°C |
| 133 | 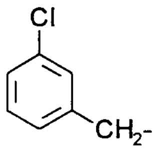 | 173°C |
| 134 | 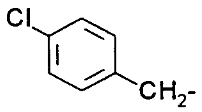 | 166-167°C |
| 135 | 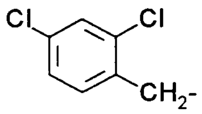 | 182°C |
| 136 | 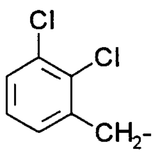 | 176°C |
| 137 | 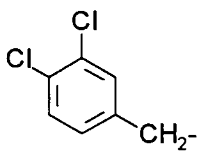 | |
| 138 | 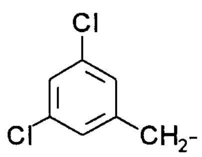 | |
| 139 | 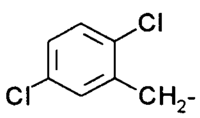 | |
| 140 | 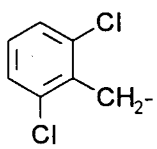 | |

| Bsp.-Nr. | $R^4$ | Fp. |
|---|---|---|
| 141 | 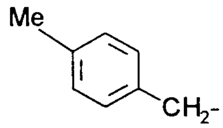 | 186°C |
| 142 | 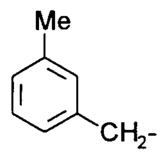 | |
| 143 | 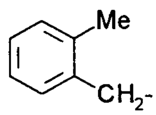 | |
| 144 | 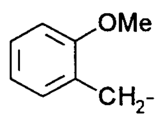 | |
| 145 | 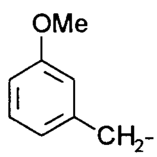 | |
| 146 | 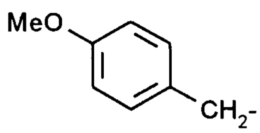 | 160°C |
| 147 | 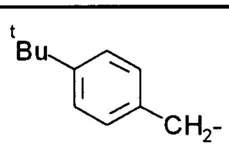 | >200°C |
| 148 | 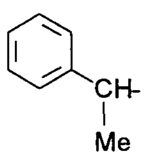 | 161-163°C |
| 149 | 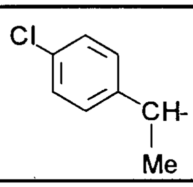 | |
| 150 | 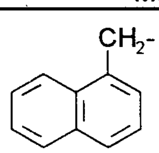 | 169-170°C |

| Bsp.-Nr. | $R^4$ | Fp. |
|---|---|---|
| 151 | 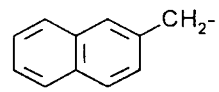 | |
| 152 | 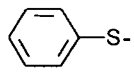 | 213-214°C |
| 153 | 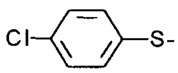 | 207-208°C |
| 154 | 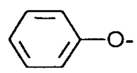 | 204°C |
| 155 | 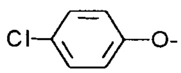 | |
| 156 | 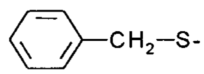 | 177-178°C |
| 157 | 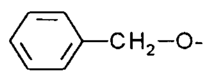 | 214°C |
| 158 | 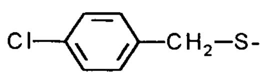 | |
| 159 | 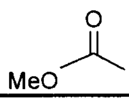 | |
| 160 | 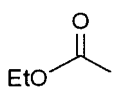 | 187°C |
| 161 | 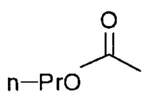 | |
| 162 | 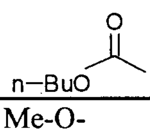 | |
| 163 | Me-O- | 149-150°C |
| 164 | Et-O- | |
| 165 | n-Pr-O- | |
| 166 | n-Bu-O- | |

| Bsp.-Nr. | $R^4$ | Fp. |
|---|---|---|
| 167 | Me-S-$CH_2$- | |
| 168 | Et-S-$CH_2$- | |
| 169 | n-Pr-S-$CH_2$- | |
| 170 | n-Bu-S-$CH_2$- | |
| 171 | i-Pr-S-$CH_2$- | |
| 172 | n-$C_5H_4$-S-$CH_2$- | |
| 173 | Me-S-$(CH_2)_2$- | |
| 174 | Et-S-$(CH_2)_2$- | |
| 175 | n-Pr-S-$(CH_2)_2$- | |
| 176 | n-Bu-S-$(CH_2)_2$- | |
| 177 | Me-O-$CH_2$- | |
| 178 | Et-O-$CH_2$- | |
| 179 | n-Pr-O-$CH_2$- | |
| 180 | n-Bu-O-$CH_2$- | |
| 181 | n-Pr-O-$CH_2$- | |
| 182 | n-$C_5H_4$-O-$CH_2$- | |
| 183 | Me-O-$(CH_2)_2$- | |
| 184 | Et-O-$(CH_2)_2$- | |
| 185 | n-Pr-O-$(CH_2)_2$- | |
| 186 | n-Bu-O-$(CH_2)_2$- | |
| 187 | Ph-S-$CH_2$- | |
| 188 | Ph-O-$CH_2$- | |
| 189 | Ph-$CH_2$-S-$CH_2$- | |
| 190 | PH-$CH_2$-O-$CH_2$- | |
| 191 | 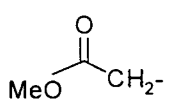 | |
| 192 | 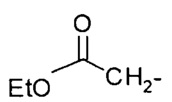 | 135-136°C |
| 193 | 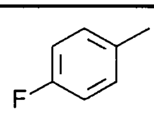 | 145-6°C |
| 194 | 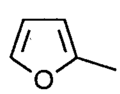 | |

| Bsp.-Nr. | $R^4$ | Fp. |
|---|---|---|
| 195 | 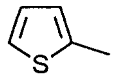 | |
| 196 | 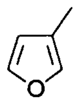 | |
| 197 | 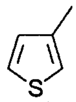 | |
| 198 | 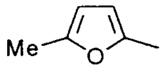 | |
| 199 | 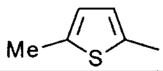 | |
| 200 | 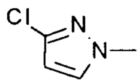 | 200°C Zers. |
| 201 | 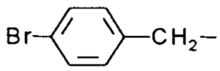 | 170-171°C |
| 202 | 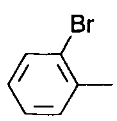 | 162-163 |
| 203 | 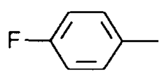 | 174-175°C |
| 204 | 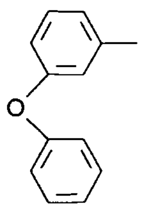 | 148-149 |
| 205 | n-$C_{17}$-$H_{39}$ | 150-154°C |
| 206 | 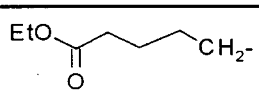 | |
| 207 | 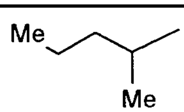 | |

Beispiel 4 aus Tabelle 2 (2/004)
(3-monosubstituierte Verbindungen)

4,6 g (0,05 Mol) Thiosemicarbazid und 7,9 g (0,05 Mol) Butyrylessigsäureethylester werden zusammen mit 100 ml Ethanol 3 Stunden auf Rückflußtemperatur gehalten. Dann läßt man auf Raumtemperatur abkühlen und gibt unter Rühren portionsweise 5,9 g (0,05 Mol, 97 %ig) Kalium-tert.-butylat zu, um danach 4 Stunden bei Raumtemperatur weiterzurühren. Anschließend rührt man den Kolbeninhalt in ein Gemisch aus 800 ml Wasser/50 ml conc. HCl ein und saugt den ausgefallenen Niederschlag ab. Nach gründlichem Waschen mit Wasser wird das feuchte Produkt im Trockenschrank (50 mbar/60 °C) bis zur Gewichtskonstanz belassen.

Ausbeute: 6,9 g (74,5 % d. Th.)

Fp.: 160-161 °C farbloser Feststoff.

In gleicher Weise können aus den entsprechenden Acylessigsäureethylestern die in der nachstehenden Tabelle 2 aufgeführten, in 3-Stellung substituierten 1-Thiocarbamoyl-5-hydroxy-pyrazole erhalten werden.

Die Charakterisierung der anfallenden Pyrazole erfolgt durch Schmelzpunktbestimmung.

**Tabelle 2**

$R^1 = R^2 = R^4 = H$

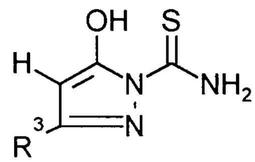

| **Bsp.-Nr.** | **$R^3$** | **Fp.** |
|---|---|---|
| 1 | H- | |
| 2 | Me- | 178°C |
| 3 | Et- | 154-158°C |
| 4 | n-Pr- | 160-161°C |
| 5 | i-Pr- | 153°C |
| 6 | c-Pr- | 158°C |
| 7 | n-Bu- | 148°C |
| 8 | i-Bu | 153°C |
| 9 | s-Bu- | 145-146°C |
| 10 | t-Bu- | |
| 11 | n-$C_5H_4$ | 149°C |
| 12 | 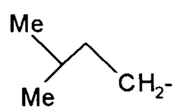 | 151°C |
| 13 | 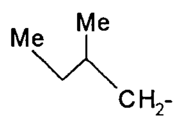 | 142°C |
| 14 | 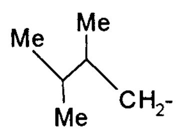 | |
| 15 | 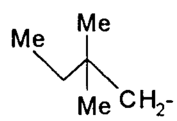 | |
| 16 | 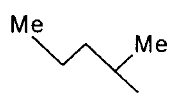 | 137°C |
| 17 | 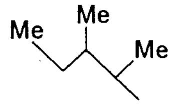 | |
| 18 | 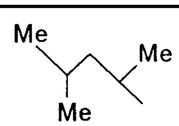 | |

| Bsp.-Nr. | $R^3$ | Fp. |
|---|---|---|
| 19 | 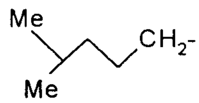 | |
| 20 | 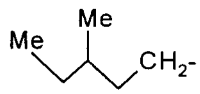 | |
| 21 | 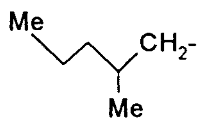 | |
| 22 | 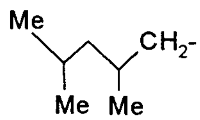 | |
| 23 | 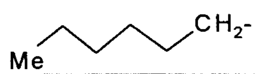 | 142-143°C |
| 24 | 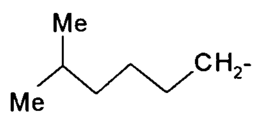 | |
| 25 | 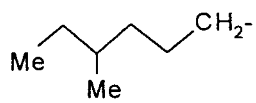 | |
| 26 | 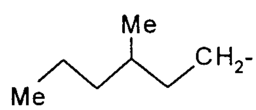 | |
| 27 | 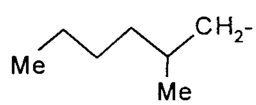 | |
| 28 | 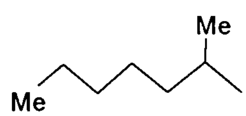 | |
| 29 | 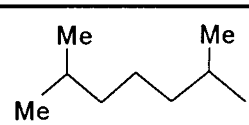 | |
| 30 | 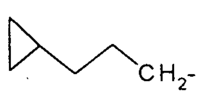 | |
| 31 | 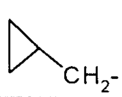 | |

| Bsp.-Nr. | $R^3$ | Fp. |
|---|---|---|
| 32 | 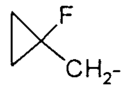 | |
| 33 | 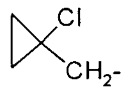 | |
| 34 | 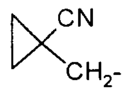 | |
| 35 | NC-$CH_2$-$CH_2$- | |
| 36 | $CF_3$- | 87-88°C |
| 37 | $CF_3$-$CF_2$- | |
| 38 | $CF_3$-$CF_2$-$CF_2$- | |
| 39 | $CF_2$-$CF_2$-$CH_2$- | |
| 40 | $CF_3$-$CF_2$-$CF_2$-$CH_2$- | |
| 41 | 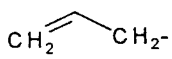 | |
| 42 | 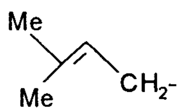 | |
| 43 | 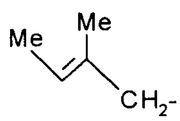 | |
| 44 | 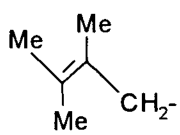 | |
| 45 | 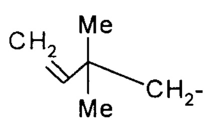 | |
| 46 | 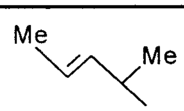 | |
| 47 | 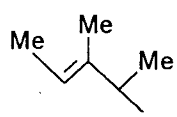 | |

| Bsp.-Nr. | $R^3$ | Fp. |
|---|---|---|
| 48 | 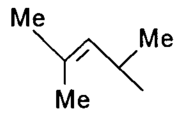 | |
| 49 | 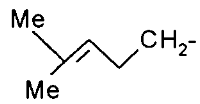 | |
| 50 | 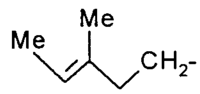 | |
| 51 | 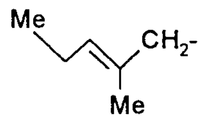 | |
| 52 | 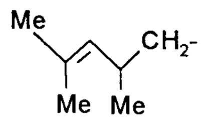 | |
| 53 | 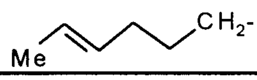 | |
| 54 | 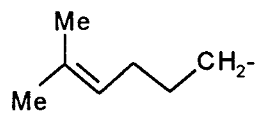 | |
| 55 | 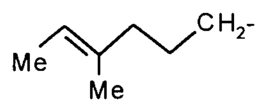 | |
| 56 | 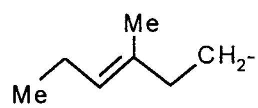 | |
| 57 | 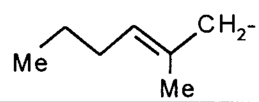 | |
| 58 | 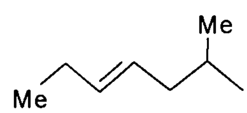 | |
| 59 | 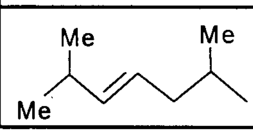 | |

| Bsp.-Nr. | $R^3$ | Fp. |
|---|---|---|
| 60 | 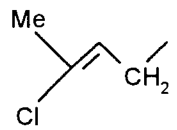 | |
| 61 | 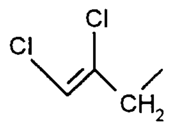 | |
| 62 | 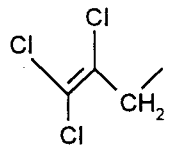 | |
| 63 | 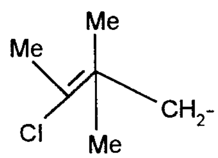 | |
| 64 | 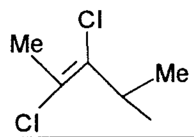 | |
| 65 | 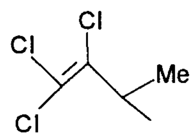 | |
| 66 | 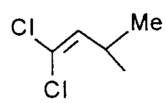 | |
| 67 | 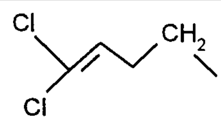 | |
| 68 | 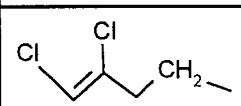 | |
| 69 | 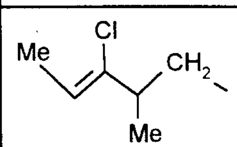 | |
| 70 | 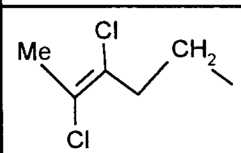 | |

| Bsp.-Nr. | $R^3$ | Fp. |
|---|---|---|
| 71 | 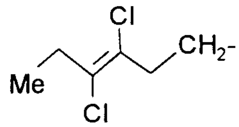 | |
| 72 | 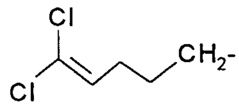 | |
| 73 | 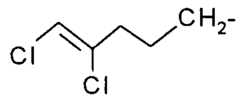 | |
| 74 | 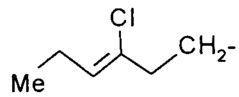 | |
| 75 | 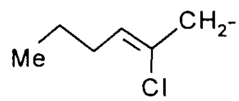 | |
| 76 | 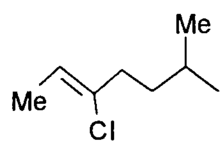 | |
| 77 | 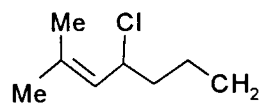 | |
| 78 | $n\text{-}C_7H_{15}$ | |
| 79 | $n\text{-}C_8H_{17}$ | 134-135°C |
| 80 | $n\text{-}C_9H_{19}$ | |
| 81 | $n\text{-}C_{10}\text{-}H_{21}$ | 154°C |
| 82 | $n\text{-}C_{11}H_{23}$ | |
| 83 | $n\text{-}C_{12}H_{25}$ | |
| 84 | $n\text{-}C_{13}H_{27}$ | |
| 85 | 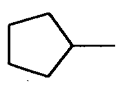 | 171°C |
| 86 | 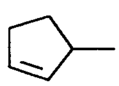 | |

| Bsp.-Nr. | $R^3$ | Fp. |
|---|---|---|
| 87 | 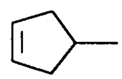 | |
| 88 | 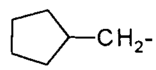 | |
| 89 | 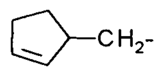 | |
| 90 | 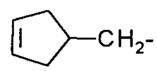 | |
| 91 | 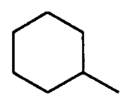 | 154°C |
| 92 | 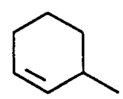 | |
| 93 | 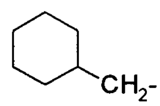 | |
| 94 | 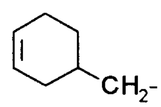 | |
| 95 | 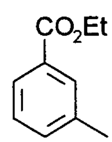 | |
| 96 | 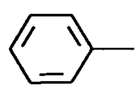 | 142-143°C |
| 97 | 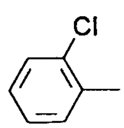 | |
| 98 | 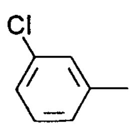 | |
| 99 | 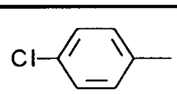 | |

| Bsp.-Nr. | $R^3$ | Fp. |
|---|---|---|
| 100 | 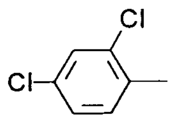 | |
| 101 | 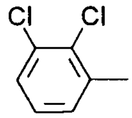 | |
| 102 | 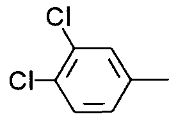 | |
| 103 | 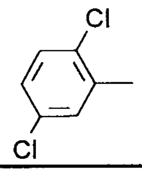 | |
| 104 | 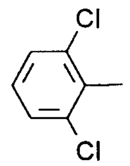 | |
| 105 | 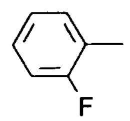 | 135-136°C |
| 106 | 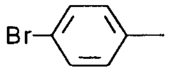 | |
| 107 | 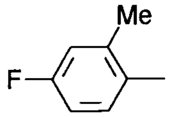 | |
| 108 | 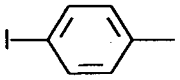 | |
| 109 | 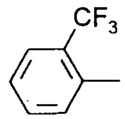 | |

| Bsp.-Nr. | $R^3$ | Fp. |
|---|---|---|
| 110 | 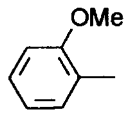 | |
| 111 | 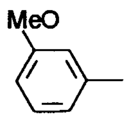 | |
| 112 | 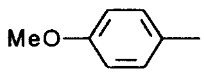 | |
| 113 | 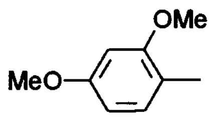 | |
| 114 | 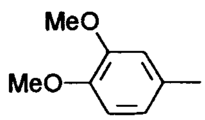 | |
| 115 | 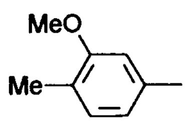 | |
| 116 | 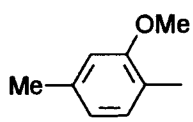 | |
| 117 | 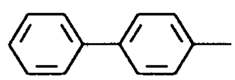 | |
| 118 | 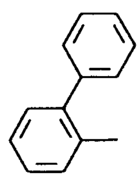 | |
| 119 | 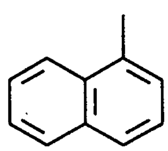 | |
| 120 | 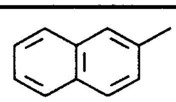 | |

| Bsp.-Nr. | $R^3$ | Fp. |
|---|---|---|
| 121 | 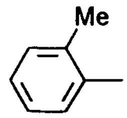 | |
| 122 | 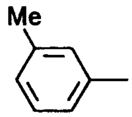 | |
| 123 | 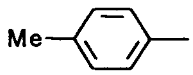 | |
| 124 | 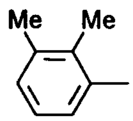 | |
| 125 | 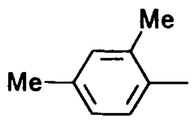 | |
| 126 | 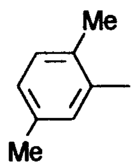 | |
| 127 | 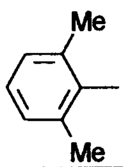 | |
| 128 | 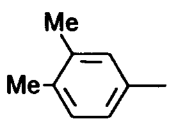 | |
| 129 | 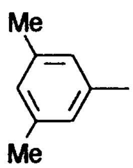 | |
| 130 | 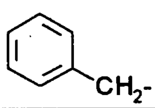 | |

| Bsp.-Nr. | $R^3$ | Fp. |
|---|---|---|
| 131 | 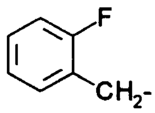 | |
| 132 | 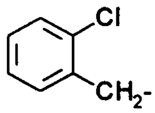 | |
| 133 | 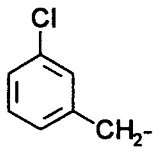 | |
| 134 | 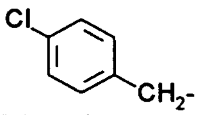 | |
| 135 | 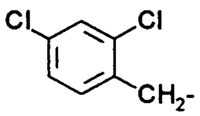 | |
| 136 | 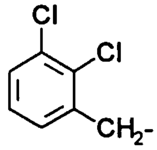 | |
| 137 | 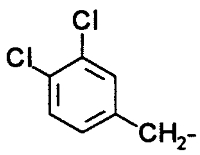 | |
| 138 | 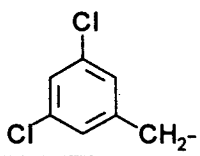 | |
| 139 | 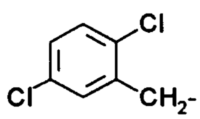 | |
| 140 | 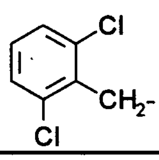 | |

| Bsp.-Nr. | $R^3$ | Fp. |
|---|---|---|
| 141 | 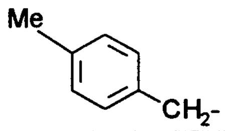 | |
| 142 | 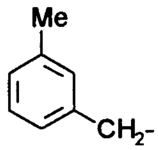 | |
| 143 | 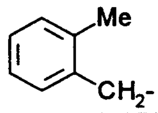 | |
| 144 | 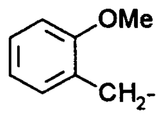 | |
| 145 | 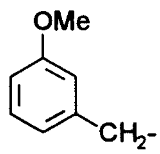 | |
| 146 | 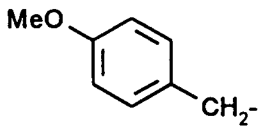 | |
| 147 | 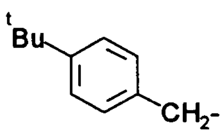 | |
| 148 | 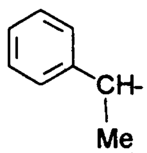 | |
| 149 | 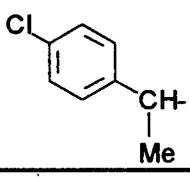 | |
| 150 | 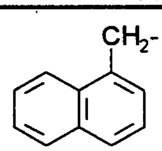 | |

| Bsp.-Nr. | $R^3$ | Fp. |
|---|---|---|
| 151 | 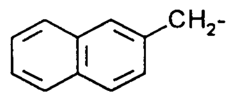 | |
| 152 | 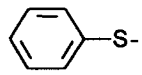 | |
| 153 | 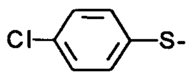 | |
| 154 | 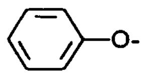 | |
| 155 | 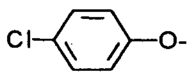 | |
| 156 | 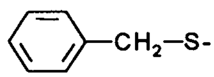 | |
| 157 | 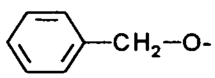 | |
| 158 | 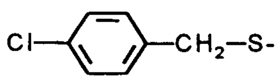 | |
| 159 | 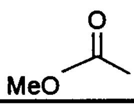 | |
| 160 | 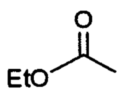 | |
| 161 | 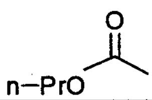 | |
| 162 | 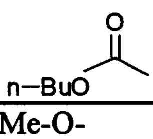 | |
| 163 | Me-O- | |
| 164 | Et-O- | |
| 165 | n-Pr-O- | |
| 166 | n-Bu-O- | |

| Bsp.-Nr. | $R^3$ | Fp. |
|---|---|---|
| 167 | Me-S-$CH_2$- | |
| 168 | Et-S-$CH_2$- | 118-119°C |
| 169 | n-Pr-S-$CH_2$- | |
| 170 | n-Bu-S-$CH_2$- | |
| 171 | i-Pr-S-$CH_2$- | |
| 172 | n-$C_5H_4$-S-$CH_2$ | |
| 173 | Me-S-$(CH_2)_2$- | |
| 174 | Et-S-$(CH_2)_2$- | |
| 175 | n-Pr-S-$(CH_2)_2$- | |
| 176 | n-Bu-S-$(CH_2)_2$- | |
| 177 | Me-O-$CH_2$- | |
| 178 | Et-O-$CH_2$- | |
| 179 | n-Pr-O-$CH_2$- | |
| 180 | n-Bu-O-$CH_2$- | |
| 181 | n-Pr-O-$CH_2$- | |
| 182 | n-$C_5H_4$-O-$CH_2$- | |
| 183 | Me-O-$(CH_2)_2$- | 140°C |
| 184 | Et-O-$(CH_2)_2$- | |
| 185 | n-Pr-O-$(CH_2)_2$- | |
| 186 | n-Bu-O-$(CH_2)_2$- | |
| 187 | Ph-S-$CH_2$- | |
| 188 | Ph-O-$CH_2$- | 178,6°C |
| 189 | Ph-$CH_2$-S-$CH_2$- | |
| 190 | PH-$CH_2$-O-$CH_2$- | |
| 191 | 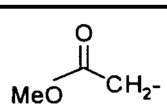 | 142°C |
| 192 | 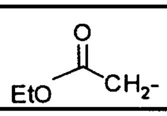 | 138°C |

| Bsp.-Nr. | $R^3$ | Fp. |
|---|---|---|
| 193 | 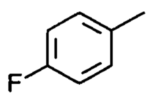 | 145-6°C |
| 194 | 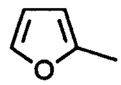 | 152°C |
| 195 | 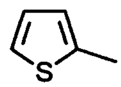 | |
| 196 | 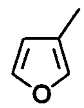 | |
| 197 | 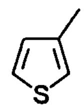 | |
| 198 | 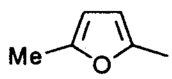 | |
| 199 | 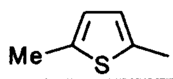 | |
| 200 | 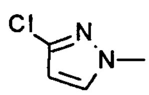 | |
| 201 | 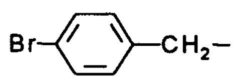 | |
| 202 | 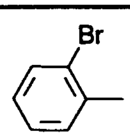 | |
| 203 | 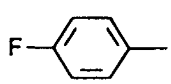 | |
| 204 | 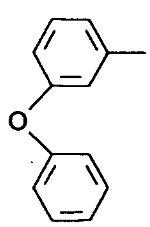 | |
| 205 | n-$C_{17}$-$H_{39}$ | |

| Bsp.-Nr. | $R^3$ | Fp. |
|---|---|---|
| 206 | 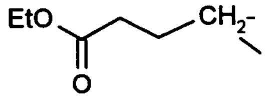 | 130°C |
| 207 | 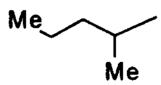 | |

Die Numerierung der nachfolgenden Beispiele folgt den vorgegangenen Tabelle 1und 2 und setzt sich aus Tabellen-Nummer/Beispiel-Nummer zusammen.

**Beispiele** (3,4-disubstituierte Verbindungen)

**Tabellen 3-8**

Beispiel 3 aus Tabelle 3

Ein Gemisch aus 3,9 g (0,025 Mol) 2-Ethyl-acetessigsäureethylester, 2,3 g (0,025 Mol) Thiosemicarbazid und 100 ml Ethanol wird unter Rühren 3 Stunden auf Rückflußtemperatur gehalten. Anschließend wird das Reaktionsgemisch auf 20 °C abgekühlt und unter Rühren portionsweise mit 2,9 g (0,025 Mol; 97 %ig) Kalium-tert.-butylat versetzt. Die erhaltene Aufschlämmung wird dann 3 Stunden bei 20-30 °C gerührt, anschließend in verdünnte Salzsäure (500 ml Wasser/20 ml conc. HCl) eingerührt und der Niederschlag abgesaugt. Man wäscht gründlich mit Wasser und trocknet in der Wärme bis zur Gewichtskonstanz.

Ausbeute: 2,9 g (82,5 % d. Th.)
Fp.: 163 °C

In gleicher Weise können aus den entsprechenden α-substituierten β-Ketoestern die in der nachstehenden Tabellen 3-8 und 65, aufgeführten 3,4-disubstituierten 1-Thiocarbamoyl-5-hydroxypyrazole erhalten werden.

**Tabelle 3**

$R^1 = R^2 = H$
$R^3 = Me$
$R^4$ = s. Tabelle 1

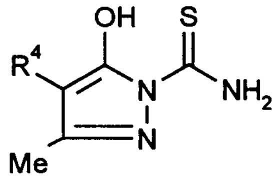

| | |
|---|---|
| 3/002 | F: 164°C |
| 3/035 · $H_2O$ | F: 153°C |
| 3/130 | F: 160°C |
| 3/148 | F: 142-143°C |
| 3/003 | F: 163°C |
| 3/007 | F: 139°C |
| 3/042 | F: 148°C |
| 3/078 | F: 124°C |
| 3/083 | F: 112°C |
| 3/023 | F: 144°C |
| 3/005 | F: 137°C |

**Tabelle 4**

$R^1 = R^2 = H$
$R^3$ = Tabelle 1
$R^4 = Me$

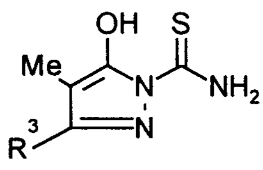

| | |
|---|---|
| 4/003 | F: 152-153°C |
| 4/004 | F: 123°C |
| 4/007 | F: 131°C |
| 4/011 | F: 120°C |

Tabelle 5= Tabelle 3nur $R^3$ = Et

Tabelle 6= Tabelle 3nur $R^3$ = n-Pr

Tabelle 7 = Tabelle 4nur $R^4$ = Et

Tabelle 8 = Tabelle 4nur $R^4$ = n-Pr

Tabellen 9 bis 16analog 1 - 8nur $R^1$ = Me

Tabellen 17 bis 32analog 1 - 8nur $R^1$ = $R^2$ = Me

Tabellen 33 bis 48analog 1 - 8nur $R^1$ = Allyl

Tabellen 49 bis 64analog 1 - 8nur $R^1$ = Phenyl

| | | |
|---|---|---|
| 49/002 | F: 204-205°C | 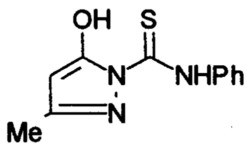 |
| 10/002 | F: 210°C Zers. | 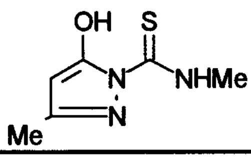 |
| 9/192 | F: 176°C | 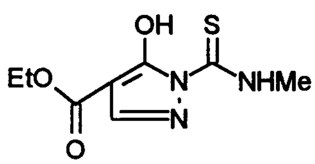 |

| | | |
|---|---|---|
| 16/003 | F: 74-75°C | 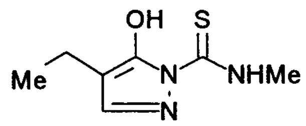 |
| 17/130 | F: 181°C | 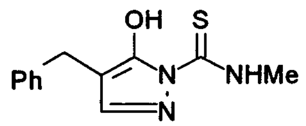 |
| 11/023 | F: 87-88°C | 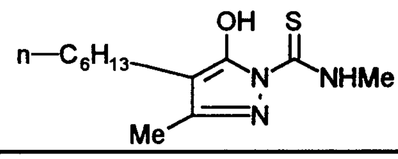 |
| 10/007 | F: 147°C | 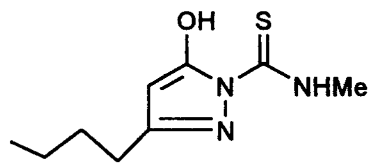 |
| 9/079 | F: 117°C | 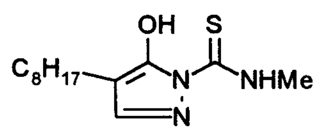 |
| 49/079 | F: 153-156°C | 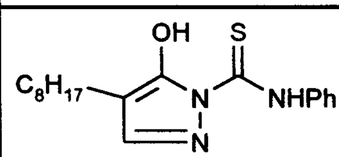 |
| 51/003 | F: 175°C | 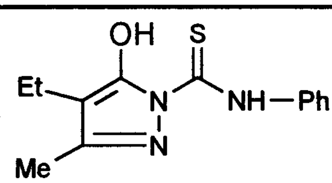 |
| 33/079 | F: 71-72°C | 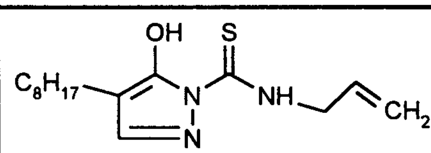 |
| 49/007 | F: 209-210°C | 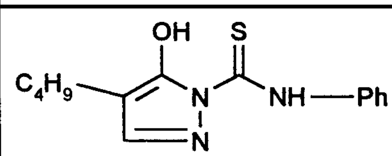 |

| | | |
|---|---|---|
| 9/007 | F: 134°C | 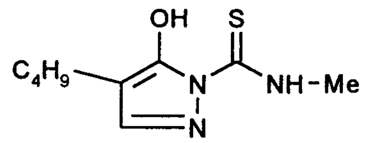 |
| 33/007 | F: 80-81°C | 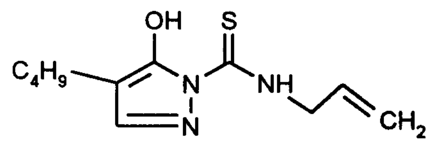 |
| 15/002 | F: 153-154°C | 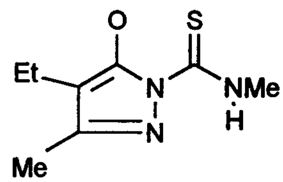 |
| 34/007 | F: 92°C | 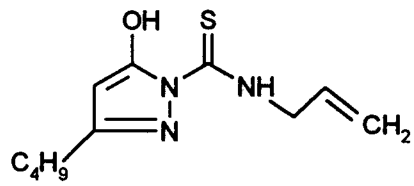 |

**Beispiele zu Tabelle 65**

Wenn $R^3$, $R^4$ eine gesättigte Kette darstellen, erfolgt Herstellung wie zu Tabellen 3 bis 8, bei ungesättigten, von Imidazolinon abgeleiteten Verbindungen wie folgt:
13,8 g 2-Chloro-3,5-Dinitrobenzoesäüreethylester, 60 ml Ethanol und 13,7 g Thiosemicarbazid werden 54 h bei 25 ° C gerührt und dann durch Absaugen der Mutterlauge 22 g

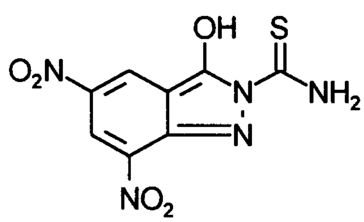

vom Schmelzpunkt 153 ° C isoliert,
oder
4,2 g Imidazolinon, 5,0 g Phenylsenföl werden 3 h in Toluol refluxiert und die beim Abkühlen ausscheidenden Kristalle isoliert; 0,6 g, F: 194 ° C Zers.

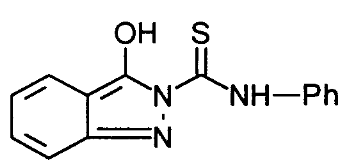

Für Verbindungen dieses Typs ist auch die Keto-Form eine besonders relevante Spezies.

**Tabelle 65**

$R^3$ und $R^4$ bilden einen Cyclus

$R^1$ und $R^2$ s. Beispiele

| | | |
|---|---|---|
| 65/001 | 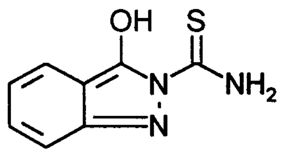 | |
| 65/002 | 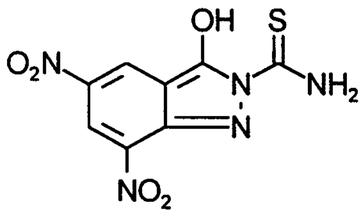 | F: 153°C |

| | | |
|---|---|---|
| 65/003 | 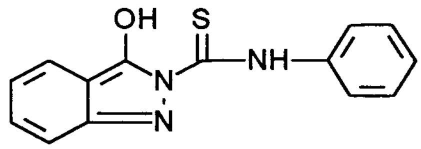 | F: 194°C (Zers.) |
| 65/004 | 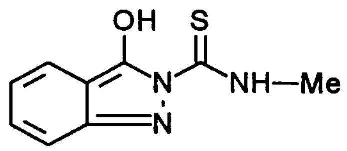 | F: 199°C |
| 65/006 | 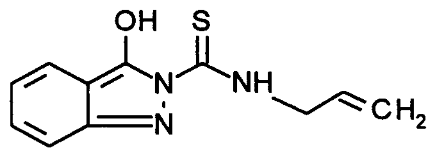 | F: 160°C |
| 65/007 | 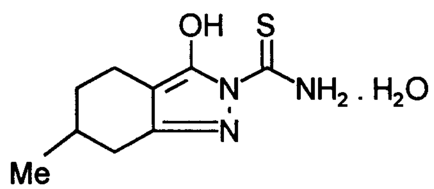 | F: 173°C |
| 65/008 | 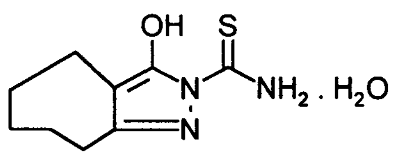 | F: 148°C |

| | | |
|---|---|---|
| 65/009 | 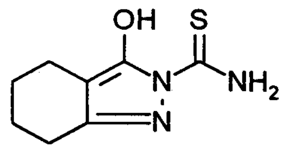 | F: 179-180°C |
| 65/010 | 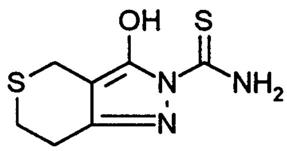 | F: 102-103°C |
| 65/011 | 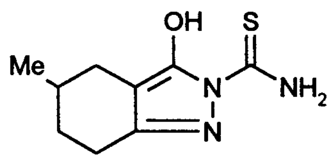 | F: 176°C |

**Tabelle 66-130**

dto. Tabelle 1-65, nur jeweils Komplexe mit Metallen s. Beispiele

| | | |
|---|---|---|
| 66/007 | · $2H_2O$ · 1/2 Zn | F: 189-192°C |
| 66/130 | · 1/2 Zn | F: 160°C |
| 66/007 | · 1/2 Zn | F: 178-180°C |

Herstellung der Verbindungen aus Tabellen 131-139:

Beispiel Nr. 131/007

10,0 g (0,05 Mol) 86 %iger α-Formyl-hexansaureethylester und 5,3 g (0,05 Mol) Thiocarbohydrazid werden in 100 ml Ethanol 3,5 Stunden refluxiert und anschließend bei 25 °C 5,8 g (0,05 Mol) Kalium-tert.-butylat zugesetzt. Nach Rühren über Nacht, Verdampfen des Lösemittels im Reaktionsverdampfer und Versetzen mit 200 ml Wasser, wird abgesaugt, das Filtrat angesaugt und erneut abgesaugt.
Man erhält 1,0g 131/007 vom Schmelzpunkt 116-118 °C.

__Tabelle 131-139__

dto. Tabelle 1-8 oder 65 nur mit folgender Grundstruktur:

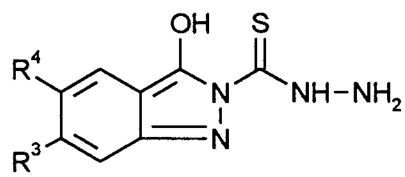

| | | | |
|---|---|---|---|
| 131/007 | F: 116-118°C | | 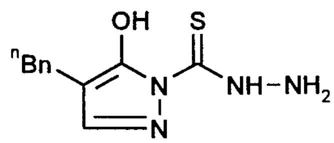 |
| 133/007 | F: 160-165°C | | 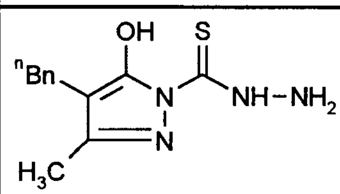 |
| 139/009 | F: 129-130°C | | 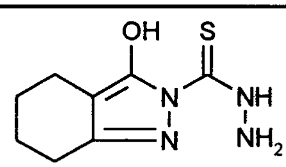 |

**Patentansprüche**

**1.** Arzneimittel enthaltend Verbindungen der allgemeinen Formel (I)

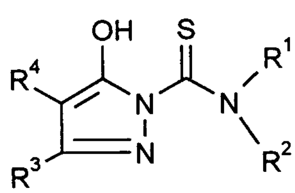

(I)

in der

$R^1$, $R^2$ unabhängig voneinander für Wasserstoff, Alkyl, Cycloalkyl, Alkenyl oder Aryl stehen, letzteres gegebenenfalls substituiert mit $C_{1-6}$-Alkyl oder Halogen

oder $R^1$ = H, $R^2$ = $NH_2$

$R^3$, $R^4$ unabhängig voneinander für Wasserstoff oder gegebenenfalls substituiertes Alkyl, Alkenyl,

Alkinyl, Cycloalkyl, Cycloalkenyl, Alkyl (Cycloalkyl), Alkenyl (Cycloalkenyl), Alkoxy, Alkylthio, Alalkoxy, Aralkylthio, Aralkyl, Aryl, Hetaryl, Aryloxy, Hetaryloxy, Arylthio, Hetarylthio, Alkoxycarbonyl, Alkoxycarbonalalkyl, Cyanoalkyl oder

$R^3$, $R^4$ bilden einen gesättigten oder teilweise oder gänzlich ungesättigten Ring, in Summe von 5 bis 9 Atomen, wovon alle bis auf maximal zwei Stickstoff-, Schwefel- oder Sauerstoffatome Kohlenstoffatome sind, oder ihre Metallkomplexe.

**2.** Arzneimittel enthaltend Verbindungen der Formel (I) gemäß Anspruch 1, worin

$R^1$ Wasserstoff oder gegebenenfalls substituiertes Alkyl, Alkenyl oder Aryl,
$R^2$ Wasserstoff
$R^3$ Wasserstoff oder gegebenenfalls substituiertes Alkyl, Cycloalkyl, Alkenyl, Aralkyl oder Aryl
$R^4$ Wasserstoff oder gegebenenfalls substituiertes Alkyl, Cycloalkyl, Alkenyl, Aralkyl oder Aryl bedeuten.

**3.** Arzneimittel enthaltend Verbindungen der Formel (I) gemäß Anspruch 1, worin

$R^1$, $R^2$ Wasserstoff
$R^3$ gegebenenfalls substituiertes Alkyl, Aralkyl oder Aryl
$R^4$ Wasserstoff, gegebenenfalls substituiertes Alkyl, Aralkyl oder Aryl bedeuten.

**4.** Verbindungen der Formel (I) gemäß Anspruch 1, worin

$R^1$und $R^2$ Wasserstoff,
$R^3$ gegebenenfalls substituiertes Alkyl oder Aralkyl
$R^4$ Wasserstoff, gegebenenfalls substituiertes Alkyl, Aralkyl
bedeuten.

**5.** Verbindungen der Formel (II)

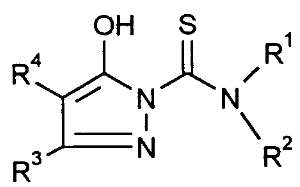

(II)

worin

$R^1$, $R^2$ H, Alkenyl oder Aryl, mit der Bedingung, daß $R^1$ und $R^2$ nicht gleichzeitig Wasserstoff sind, bedeuten und
$R^3$, $R^4$ die in den Ansprüchen 1 bis 4 zu den Verbindungen der Formel (I) angegebenen Bedeutungen aufweisen.

**6.** Verwendung der Verbindungen gemäß den Ansprüchen 1 bis 5 zur Herstellung von Arzneimitteln.

Europäisches Patentamt

# EUROPÄISCHER RECHERCHENBERICHT

Nummer der Anmeldung
EP 94 11 1858

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| X | CHEMICAL ABSTRACTS, vol. 107, no. 17, 26. Oktober 1987, Columbus, Ohio, US; abstract no. 154289r, K. WEGNER ET AL. 'H2-Antihistaminics. XXXIII. Synthesis and H2-antagonistic activity of heteroaromatic (thio)carboxamides and triazole(thi)one-derivatives of [(piperidinomethyl)phenoxy]propylamine.' Seite 698 ;Spalte 2 ; * Zusammenfassung und Chemical Abstracts, CHEMICAL SUBSTANCES, 12th Collective Index, Bd. 106-115, 1987-1991, siehe Seite 78341CS, RN [110365-94-1] und [110365-88-3] * & ARCHIV DER PHARMAZIE, Bd.320, Nr.2, 1987, WEINHEIM, DE Seiten 108 - 114 --- | 1,2 | C07D231/20 A61K31/415 C07D231/28 C07D231/48 C07D231/54 C07D405/04 C07D495/04 |
| D,A | EP-A-0 515 934 (BAYER AG) * das ganze Dokument * ----- | 1,5 | |

| RECHERCHIERTE SACHGEBIETE (Int.Cl.6) |
|---|
| C07D A61K |

Der vorliegende Recherchenbericht wurde für alle Patentansprüche erstellt

| Recherchenort | Abschlußdatum der Recherche | Prüfer |
|---|---|---|
| DEN HAAG | 22. September 1994 | Fink, D |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)